# EUROPEAN PATENT APPLICATION

(11) **EP 4 179 952 A1**
(43) Date of publication of application: **17.05.2023**
(21) Application number: 21837968.3
(22) Date of filing: 09.07.2021
(51) Int. Cl.: A61B 1/00, A61B 1/04

(54) **MAGNETIC CONTROL DEVICE AND MAGNETICALLY CONTROLLED CAPSULE ENDOSCOPE SYSTEM**

(30) Priority: 10.07.2020 CN 202010664939
(71) Applicant: Ankon Medical Technologies (Shanghai) Co., Ltd, Shanghai 201206 (CN)
(72) Inventor: HAN, Chao, Shanghai 201206 (CN); ZHANG, Shaobang, Shanghai 201206 (CN); DUAN, Xiaodong, Shanghai 201206 (CN)
(74) Representative: Luppi Intellectual Property S.r.l.
(86) International application number: PCT/CN2021/105593
(87) International publication number: WO 2022/007963

(57) **Abstract**

A magnetic control device (1) and a magnetically controlled capsule endoscope system. The magnetic control device (1) comprises a mounting base (11), a base (12), a magnetic component (16), a first motor (13), and a second motor (14). The mounting base (11) is used for hoisting the magnetic control device (1). The base (12) is connected to the mounting base (11). The magnetic component (16) comprises a magnet (161). The magnet (161) is used for driving a capsule endoscope to rotate. The first motor (13) is used for driving the magnetic component (16) to rotate around a first axis (L1). The first motor (13) penetrates through the mounting base (11) and is mounted on the base (12) along a first direction of the magnetic control device (1). The second motor (14) is used for driving the magnetic component (16) to rotate around a second axis (L2). The second motor (14) is mounted on the base (12). The first axis (L1) is intersected with the second axis (L2). The mounting space of the magnetic control device (1) is reasonably controlled, so that the magnetic control device (1) is more compact and smaller in structure and convenient to move.

## Description

The application claims priority to Chinese patent Application No. 202010664939.3, filed July10, 2020, titled "Magnetic Control Device and Magnetically Controlled Capsule Endoscope System", all of which are incorporated herein by reference in their entirety.

### TECHNICAL FIELD

The present disclosure relates to the technical field of capsule endoscope control system, and in particular to a magnetic control device and a magnetically controlled capsule endoscope system.

### BACKGROUND

At present, capsule endoscopy is a relatively advanced diagnostic and therapeutic means on the market for routine examination of human digestive tract. In contrast to traditional intubation type endoscope, swallowing a capsule endoscope does not cause physical and psychological discomfort to a patient, and also reduce the possibility of cross-infection.

Magnetically controlled capsule endoscope system is a capsule endoscope system that enables an actively control of the examination field of view through an operation terminal. A common method is to place a magnetic device in vitro. A magnet built in the magnetically controlled capsule endoscope system drives the capsule endoscope to move in vivo under the influence of the variation of the external magnetic field so as to realize an active adjustment of the examination field of view of the capsule endoscope. However, a current magnet control device is large and bulky, and requires a lot of space.

### SUMMARY

The present disclosure provides a magnetic control device which has characteristics of a simple structure and a small size.

An embodiment of the present disclosure provides a magnetic control device for controlling a capsule endoscope, and the magnetic control device includes a mounting base, a base, a magnetic component, a first motor, and a second motor.

The mounting base is configured to hoist the magnetic control device.

The base is connected to the mounting base.

The magnetic component includes a magnet configured to drive the capsule endoscope to rotate.

The first motor is configured to control the magnetic component to rotate around a first axis. The first motor penetrates through the mounting base and is installed at the base along a height direction of the magnetic control device.

The second motor is configured to control the magnetic component to rotate around a second axis. The second motor is installed at the base.

The first axis intersects the second axis.

In an embodiment, the base includes an upper base and a lower base.

The upper base is provided between the mounting base and the lower base along a first direction of the magnetic control device, and a space between the mounting base and the lower base defines a mounting space.

In an embodiment, an outer edge of the lower base exceeds an outer edge of the upper base.

In an embodiment, the first motor is installed at the upper base, and the second motor is connected to the lower base and is provided in the mounting space between the mounting base and the lower base.

In an embodiment, the magnetic control device further includes a mounting plate connected to the base.

At least part of the mounting plate extends to the mounting space, and the second motor is connected to the base through the mounting plate.

In an embodiment, the magnetic control device further includes a conductive slip ring including an upper slip ring and a lower slip ring.

The upper slip ring is installed at the upper base and is configured to be electrically connected to a power supply, and the lower slip ring is installed at the lower base and is configured to be electrically connected to the second motor.

In an embodiment, the conductive slip ring is a printed circuit board (PCB) type slip ring, and a thickness of the PCB type slip ring is less than or equal to 10mm.

In an embodiment, the magnetic component further includes a housing, and the magnet is fixed in the housing.

The housing includes a left housing and a right housing. The left housing is connected to the right housing through a flange, and the magnet is fixedly provided in the housing.

In an embodiment, two connecting plates are provided at two ends of the lower base, respectively, along a second direction of the magnetic control device, and the two connecting plates extend away from the upper base along the first direction of the magnetic control device.

The housing is located between the two connecting plates and is rotatably connected to the two connecting plates, and the housing rotates as driven by the first motor and the second motor.

In an embodiment, the magnetic control device further includes at least one rotation origin detection mechanism for determining whether the magnetic component is at a rotation origin.

The rotation origin detection mechanism includes a switch assembly electrically connected to the first motor and/or the second motor, and a switch mating member cooperating with the switch assembly to control an on/off of a circuit.

In an embodiment, the rotation origin detection mechanism is installed between the upper base and the lower base, the switch assembly is installed at one of the upper base and the lower base, and the switching mating member is installed at the other one of the upper base and the lower base. The first motor is controlled to start or stop through a relative rotation between the switch assembly and the switch mating member. In an embodiment, the rotation origin detection mechanism is installed at a position where the lower base and the magnetic component are connected to each other, the switch assembly is installed at one of the lower base and the magnetic component, and the switch mating member is installed at the other one of the lower base and the magnetic component. The second motor is controlled to start or stop through the relative rotation between the switch assembly and the switch mating member.

In an embodiment, the switch assembly is a photoelectric switch, the switch mating member is a code disk, and the code disk is provided with a detection portion.

Along with a relative rotation between the photoelectric switch and the code disk, when the photoelectric switch detects the detection portion, the photoelectric switch controls the circuit to be disconnected, and the magnetic component stops at the rotation origin.

In an embodiment, the switch assembly is a microswitch group, and the switch mating member is a contact mating portion.

Along with a relative rotation between the microswitch group and the contact mating portion, when the microswitch detects the contact mating portion, the microswitch group controls the circuit to be disconnected, and the magnetic component stops at the rotation origin.

In an embodiment, the first motor and/or the second motor is/are a direct current motor or a stepper motor.

A magnetically controlled capsule endoscope system according to an embodiment of the present disclosure includes the magnetic control device. The magnetic control device is carried on the magnetically controlled capsule endoscope system through the mounting base.

The technical solution according to the present disclosure can achieve the following beneficial effects.

An area covered by projection of the mounting base defines a mounting space, the second motor is located in the mounting space. The first motor and the second motor are installed by reasonably using the mounting space, thereby achieving a more compact and smaller structure and facilitating moving the magnetic control device.

It should be understood that the above description and the details to be set forth in the following text are only exemplary, but not intended to limit the present disclosure.

### BRIEF DESCRIPTION OF DRAWINGS

Fig.1 is a structural schematic diagram of a magnetic control device according to an embodiment of the present disclosure;
Fig. 2 is an enlarged view of a portion A in Fig. 1;
Fig. 3 is a section view of Fig. 1;
Fig. 4 is an enlarged view of a portion B in Fig. 3;
Fig. 5 is an enlarged view of a portion C in Fig. 3;
Fig. 6 is a section view of Fig. 1 in another direction; and
Fig. 7 is an enlarged view of a portion D in Fig. 6.

Reference signs:
1-magnetic control device;
   11-mounting base;
   111-mounting space;
12-base;
   121-upper base;
   1 22-lower base;
      122a-connecting plates;
13-first motor;
   131-first coupling;
   132-connecting shaft;
   133-spindle;
   1 34-bearing;
   135-locking nut;
   136-shaft sleeve;
14-second motor;
   141-second coupling;
   142-first timing belt pulley;
   143-timing belt;
   144-second timing belt pulley;
15-mounting plate;
16-magnetic component;
   161-magnet;
   1 62-housing;
      162a-left housing;
      162b-right housing;
      162c-flange;
      162d-shaft portion;
18-photoelectric switch;
   181-first photoelectric switch;
   181a-first groove;
   182-second photoelectric switch;
   182a-second groove;
19-code disk;
   191-first code disk;
   192-second code disk;
      192a-opening;
20-conductive slip ring;
   21-upper slip ring;
   22-lower slip ring.

The drawings herein are incorporated in and constitute a part of the description for illustrating the embodiments consistent with the present disclosure and are used together with the description to explain the principles of the present disclosure.

### DESCRIPTION OF EMBODIMENTS

In order to understand the technical solution of the present disclosure better, the embodiments according to the present disclosure are described in detail below in combination with the drawings.

It should be understood that the described embodiments are only part of the embodiments of the present disclosure. Based on the embodiments in the present disclosure, all other embodiments obtained by those skilled in the art without any creative efforts belong to the protection scope of the present disclosure.

The present disclosure is further described in detail below through specific embodiments with reference to the accompanying drawings.

At present, a common means for controlling a "capsule endoscope" is to place a magnet in vitro to cause an orderly changing magnetic field around the magnet by changing the orientation and posture of the magnet. A magnet built in a magnetically controlled capsule endoscope drives the capsule endoscope to move, so as to achieve a variation of the examination field of view of the capsule endoscope. However, a magnetic control device installed with the magnet is bulky and heavy.

In an embodiment, the magnetic control device includes a support base, a vertical rotation motor, a horizontal rotation motor, a magnetic ball and a magnetic ball mounting bracket. The support base is configured to hoist the magnetic control device, the vertical rotation motor and the horizontal rotation motor are generally installed at the left and right sides of the support base, the magnetic ball is installed at the magnetic ball mounting bracket, and the magnetic ball mounting bracket is installed below the support bracket and is rotationally connected to the support base through a bearing. The vertical rotation motor and the horizontal rotation motor control the magnetic ball mounting bracket to rotate to drive the magnetic ball to rotate, thereby causing the magnetic field around the magnetic ball to change orderly and further controlling the motion of the capsule endoscope in vivo. The support base is used for fixing the magnetic control device, and is large in size. The vertical rotation motor and the horizontal rotation motor are installed at two sides of the support base, and a transmission chain between the vertical rotation motor and/or the horizontal rotation motor and the magnetic ball is long, so that the overall size of the magnetic control device is so large that a large enough working space is required for placing the magnetic control device, and the large size of the magnetic control device makes it difficult to move the magnetic control device.

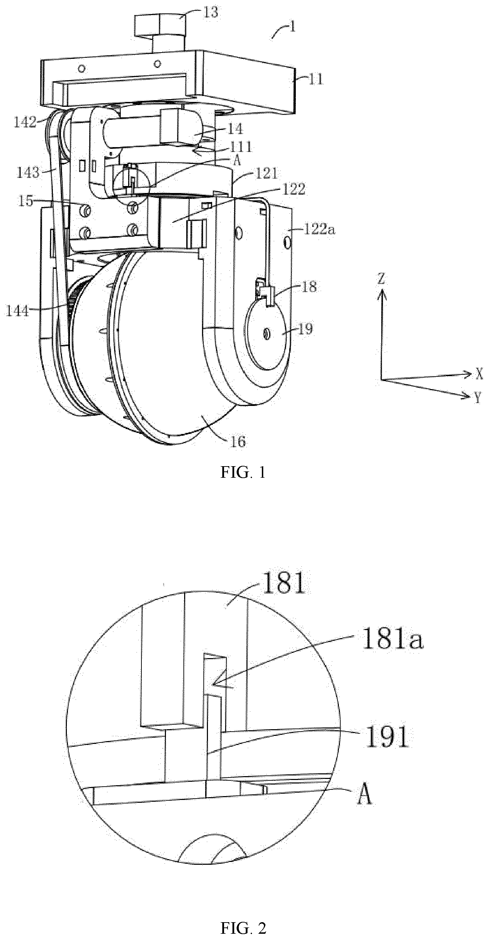

As shown in Figs. 1 to 7, in order to solve the above technical problem, a magnetically controlled capsule endoscope system according to an embodiment of the present disclosure includes a magnetic control device 1. The magnetically controlled capsule endoscope system is configured to control a capsule endoscope ingested into a human body to move so as to completely observe the gastrointestinal tract of a human body and make a diagnosis. The magnetic control device 1 includes a mounting base 11, a base 12, a magnetic component 16, a first motor 13 and a second motor 14. The mounting base 11 is configured to hoist the magnetic control device 1, the magnetic control device 1 is carried on a bracket of the capsule endoscope system through the mounting base 11, and the magnetic control device1 is fixed to the bracket through the mounting base 11. The bracket refers to an external device for installing the magnetic control device 1, which is usually installed on the ground and can drive the magnetic control device 1 to move back and forth, up and down, and left and right, so as to correspondingly move to a position close to a patient for test. The base 12 is fixedly connected to a bottom of the mounting base 11, and the magnetic component 16 includes a magnet 161 configured to drive the capsule endoscope to rotate. The first motor 13 is configured to control the magnetic component 16 to rotate around a first axis L1, and the first motor 13 penetrates through the mounting base 11 and is installed at the base 12 along a first direction of the magnetic control device 1. The second motor 14 is configured to control the magnetic component 16 to rotate around the second axis L2 and is installed at the base 12. The first axis L1 intersects the second axis L2.

In an embodiment, the first direction of the magnetic control device 1 can be defined as a height direction Z of the magnetic control device 1 (as shown in Fig. 1). In the following embodiments, the first direction is exemplarily defined as the height direction Z.

In an embodiment, the mounting base 11 is provided at the magnetic control device 1. The base 12 is fixedly installed below the mounting base 11, and the first motor 13 penetrates through the mounting base 11 and is installed at the base 12. The first motor 13 and the second motor 14 are installed by reasonably using a mounting space 111, thereby achieving a more compact and smaller structure and facilitate moving the magnetic control device 1.

The first motor 13 and the second motor 14 may be a direct current motor or a stepper motor. In the present disclosure, a direct current motor or a stepper motor is adopted instead of a servo motor, so that the magnetic control device 1 does not need an encoder and/or a reducer, and the first motor 13 and/or the second motor 14 are driven by timing belt pulleys, so that the requirements for torque of the motors is reduced, and thereby the overall size of the magnetic control device 1 is reduced. Alternatively, other motors that can drive the magnetic component 16 to rotate with high precision and that are relatively small in size may also be adopted.

In an embodiment, as shown in Figs. 1, 3 and 6, the base 12 includes an upper base 121 and a lower base 122, the first motor 13 is installed at the upper base 121, and the second motor 14 is connected to the lower base 122. Along the height direction Z of the magnetic control device 1, the upper base 121 is located between the mounting base 11 and the lower base 122. Along the height direction Z of the magnetic control device 1, a space between the mounting base 11 and the lower base 122 is defined as a mounting space 111, and an outer edge of the lower base 122 exceeds an outer edge of the upper base 121.

In this embodiment, the outer edge of the lower base 122 exceeds the outer edge of the upper base 121, so as to define a redundant mounting space 111 between the mounting base 11 and the lower base 122 besides the upper base 121. Further, the second motor 14 can be arranged in the mounting space 111 and be installed at the lower base 122, thereby achieving a more compact overall volume and effectively improving the space utilization rate of the magnetic control device 1. After being installed, the base 12, the first motor 13 and the second motor 14 do not exceed the range covered by the projection of the mounting base 1, so that the magnetic control device 1 has the characteristics of a compact structure and a small size. In an embodiment, the second axis L2 around which the second motor 14 controls the magnetic component 16 to rotate is perpendicular to the first axis L1 around which the first motor 13 controls the magnetic component 16 to rotate. At this time, the rotating shaft of the first motor 13 is in the vertical direction, and the rotating shaft of the second motor 14 is in the horizontal direction. In contrast to the prior art in which the first motor 13 and the second motor 14 are both vertically installed at the base 12, the first motor 13 and the second motor 14 in this embodiment are installed in such a way that the mounting space is greatly saved and the size of the magnetic control device 1 can be further reduced.

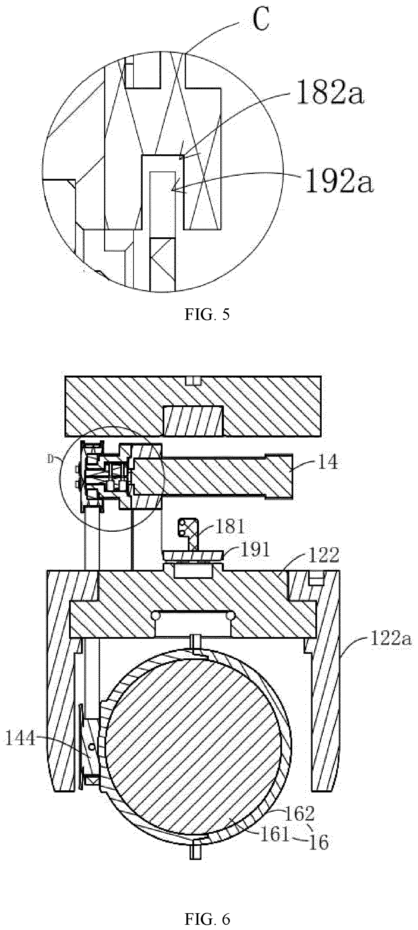

In an embodiment, as shown in Figs. 1 and 6, the magnetic control device 1 further includes a mounting plate 15 connected to the base 12. Along the height direction Z of the magnetic control device 1, at least part of the mounting plate 15 extends to the mounting space 111. The second motor 14 is connected to the base 12 through the mounting plate 15. The second motor 14 is connected to the lower base 122 through the mounting plate 15, thereby simplifying the overall structure of the lower base 122.

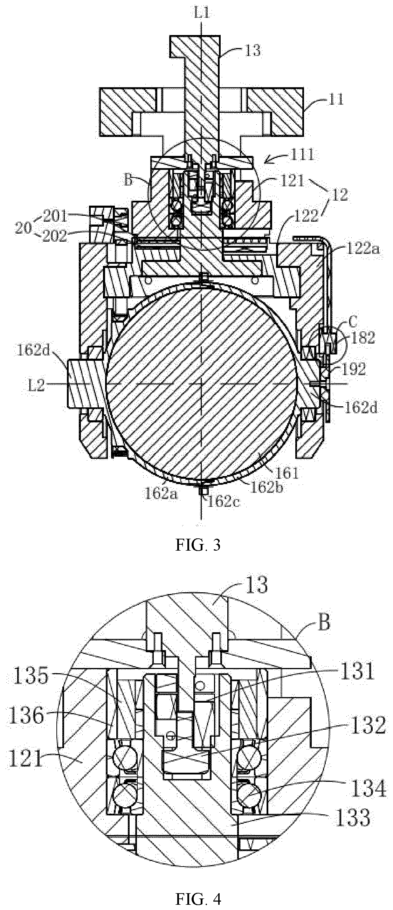

Further, as shown in Fig. 3, a conductive slip ring 20 is installed between the upper base 121 and the lower base 122. The conductive slip ring 20 is configured to conduct electricity for the second motor 14.The conductive slip ring 20 includes an upper slip ring 201 and a lower slip ring 202. The upper slip ring 201 and the lower slip ring 202 can ensure electrical connection there between while rotating relatively. The upper slip ring 201 is installed at the upper base 121 and the lower slip ring 202 is installed at the lower base 122. The upper slip ring 201 is electrically connected to a power supply (not shown), and the lower slip ring 202 can be electrically connected to the second motor 14. In a working state, the upper slip ring 201 can conduct electricity to the lower slip ring 202, so as to prevent the second motor 14 from being connected to the power supply through a long wire, and the wire may be twisted with the rotation of the lower base 122. It should be noted that the above power supply may be a power supply of the magnetic control device1 itself in the present disclosure (including a power supply at the magnetic control device 1), or may be an external power supply on a device for assembling the magnetic control device 1, and alternatively, the magnetic control device 1 maybe connected to commercial electric supply, which will not be described here.

The conductive slip ring 20 may be a PCB type slip ring, and a thickness of the PCB type slip ring is less than or equal to 10mm. Compared with other types of conductive slip rings, the PCB type slip ring has a smaller thickness, can reduce the structure in the height direction Z, and is favorable to compacting the overall size of the magnetic control device 1.

In an embodiment, as shown in Fig. 3, the magnetic component 16 further includes a housing 162, and the magnet 161 is secured within the housing 162. The housing 162 includes a left housing 162a and a right housing 162b, and the left housing 162a and the right housing 162b are connected to each other by a flange 162c. In this embodiment, the left housing 162a and the right housing 162b are fixedly connected to each other by the flange 162c. In an embodiment, the left housing 162a is fixed to a flange, which can be formed integrally with the left housing 162a, the right housing 162b is fixed to another flange, which can be formed integrally with the right housing 162b, then a flange gasket is placed between the two flanges, and finally the two flanges are tighten by bolts to be tightly combined, such that the left housing162a and the right housing 162b are fixedly connected to each other. Compared with providing a plurality of threaded holes in peripheral edges of the left housing 162a and the right housing 162b, the left housing 162a and the right housing 162b are fixedly connected more convenient and firm through being tightened through a plurality of screws in sequence. In addition, in the embodiment of the present disclosure, it is not necessary to drill threaded holes at the housing 162 to ensure the sealing performance in the housing 162, and at least four positions are required for threaded connection between the left housing 162a and the right housing 162b, which is cumbersome to install and remove. In contrast, the flange 162c makes the connection, the installation and the disassembly more convenient. Further, the connection strength between the left housing 162a and the right housing 162b is high, and the sealing performance in the housing 162 is good.

In an embodiment, as shown in FIG. 3, along a second direction Y of the magnetic control device 1, two connecting plates 122a are provided at two ends of the lower base 122, respectively, and along the height direction Z of the magnetic control device 1, the two connecting plates 122a extend away from the upper base 121. The housing 162 is located between the two connecting plates 122a, and is rotatably connected to the two connecting plates 122a. The housing 162 can rotate driven of the first motor 13 and the second motor 14. In this embodiment, the housing 162 drives the magnet 161 inside to rotate, so as to make the magnetic field around the magnet 161 change. A small magnet is built in the capsule endoscope in vivo, and the small magnet drives the capsule endoscope to move under the influence of the variation of the external magnetic field, so as to achieve the variation of the examination field of view of the capsule endoscope. Moreover, the connecting plate 122a extends in a direction away from the upper base 121, and the housing 162 is connected to the connecting plate 122a. Accordingly, the magnet 161 is away from the first motor 13 and the second motor 14, thereby reducing the influence of magnetic field interference on the first motor 13 and the second motor 14.

In an embodiment, when the first motor 13 is driven to work, the first motor 13 drives the lower base 122 to rotate through a rotating shaft thereof, and the lower base 122 drives the housing 162 to rotate through the connecting plates 122a, so that the housing 162 drives the magnet 161 to rotate along with the rotating shaft of the first motor 13, thereby changing the magnetic field in which the capsule endoscope is located, as well as the examination field of view of the capsule endoscope. When the second motor 14 is driven to work, the second motor 14 drives a drive assembly to rotate through a rotating shaft thereof, and the drive assembly is connected to the housing 162, so that the housing 162 drives the magnet 161 to rotate along with the rotating shaft of the second motor 14, thereby changing the magnetic field in which the capsule endoscope is located, as well as the examination field of view of the capsule endoscope.

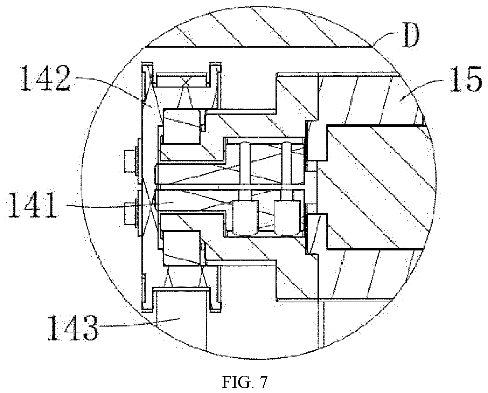

In an embodiment, as shown in Figs. 6 and 7, the drive assembly includes a first timing belt pulley 142, a timing belt 143 and a second timing belt pulley 144. The first timing belt pulley 142 is connected to the rotating shaft of the second motor 14 through a second coupling 141, and the second timing belt pulley 144 is fixedly connected to the left housing 162a (or the right housing 162b). The first timing belt pulley 142 and the second timing belt pulley 144 rotate synchronously through the timing belt 143 to drive the housing 162 to rotate, thereby making the magnet 161 to rotate.

The second direction of the magnetic control device 1 can be defined as the length direction Y of the magnetic control device 1 (as shown in Fig. 1). In the follow embodiments, the second direction is defined as the length direction Y as an example.

In an embodiment, the left housing 162a and the right housing 162b may also be connected by bolts. Alternatively, the left housing 162a and the right housing 162b may be fixedly connected by other manners, so that the left housing 162a and the right housing 162b can be fixedly connected to each other.

Further, as shown in Figs. 1, 3 and 4, there is a space between the upper base 121 and the lower base 122. The rotating shaft of the first motor 13 is connected to one end of a connecting shaft 132 through a first coupling 131, the other end of the connecting shaft 132 is installed and snapped at a spindle 133, and the spindle 133 extends to be connected to the lower base 122 along the height direction Z of the magnetic control device 1, so that the first motor 13 controls the lower base 122 to rotate. The housing 162 is installed at the lower base 122 through the connecting plate 122a, so that the first motor 13 drives the housing 162 to rotate. The first motor 13 is connected to the spindle 133 through the first coupling 131 and the connecting shaft 132 transmits torque, so that the rotation of the first motor 13 transmits the torque to the spindle 133 to reduce the excessive load on the first motor 13 and thereby protect the first motor 13.

As shown in Figs. 6 and 7, the second motor 14 is installed at the lower base 122, and the rotating shaft of the second motor 14 drives the housing 162 to rotate through the drive assembly. The rotation direction of the housing 162 controlled by the first motor 13 is perpendicular to the rotation direction of the housing 162 controlled by the first motor 14.

In an embodiment, as shown in Figs. 3 and 4, a bearing 134 and a locking nut 135 are sleeved on the spindle 133. The spindle 133 penetrates through the upper base 121, is rotatably connected to the upper base 121 through the bearing 134, and is locked by two locking nuts 135 to prevent the spindle 133 from moving when the magnetic control device 1 is inverted. A shaft sleeve 136 is sleeved outside the locking nut 135 to prevent the spindle 133 from being deviated by vibration.

In an embodiment, in order to facilitate subsequent operations for an operator after the magnetic control device 1 is powered on and initialized, the magnetic control device 1 further includes at least one rotation origin detection mechanism for determining whether the magnetic component 16 is at a rotation origin (i.e., the rotation origin of the magnet 161). The rotation origin detection mechanism at least includes a switch assembly electrically connected to the first motor 13 and/or the second motor 14, and a switch mating member cooperated with the switch assembly to control the on/off of a circuit.

It should be noted that, in this embodiment, the rotation origin may be the rotation origin (also referred to as a first zero point) of the magnetic component 16 around the first axis L1, or the rotation origin (also referred to as a second zero point) of the magnetic component 16 around the axis L2. In some cases, it may be considered that when the magnetic component 16 is at the first zero point, the N pole of the magnet 161 is facing upward and the S pole of the magnet 161 is facing downward. In other cases, other postures of the magnetic component 16 may be defined as the zero-point position, for example, the S pole of the magnet 161 is facing upward, and the N pole of the magnet 161is facing downward, etc., as long as it is convenient to control the capsule endoscope.

In an embodiment, in order to detect and control whether the magnet 161 moves to the first zero point and/or the second zero point, the rotation origin detection mechanism is installed between the upper base 121 and the lower base 122, the switch assembly is installed at one of the upper base 121 and the lower base 122, and the switch mating member is installed at the other one of the upper base 121 and the lower base 122. The first motor 13 is controlled to start or stop by a relative rotation between the switch assembly and the switch mating member. In another embodiment, the rotation origin detection mechanism is embodiment at a position where the lower base 122 and the magnetic component are connected to each other, the switch assembly is installed at one of the lower base 122 and the magnetic component 16, and the switch mating member is installed at the other one of the lower base 122 and the magnetic component 16. The second motor 14 is controlled to start or stop through the relative rotation between the switch assembly and the switch mating member.

In an embodiment, the switch assembly is a photoelectric switch 18, the switch mating member is a code disk 19, and the code disk 19 is provided with a detection portion. With a relative rotation between the photoelectric switch 18 and the code disk 19, when the photoelectric switch 18 detects the detection portion, the photoelectric switch controls the circuit to be disconnected, and the magnetic component 16 stops at the rotation origin.

In an embodiment, as shown in Figs. 3 to 7, the magnetic control device 1 further includes a photoelectric switch 18 and a code disk 19. The code disk 19 can rotate relative to the photoelectric switch 18 along with the rotation of the magnet 161.When the photoelectric switch senses the corresponding detection portion, it is determined that the magnet 161 reaches the rotation origin. In an embodiment, the photoelectric switch 18 is connected to the first motor 13 or the second motor 14 to detect whether the magnet 161 is located at the rotation origin. When the magnet 161 rotates to the rotation origin, the photoelectric switch 18 controls the first motor 13 or the second motor 14 to stop working.

In this embodiment, the code disk 19 can rotate with the magnet 161, and the photoelectric switch 18 detects the rotation position of the magnet 161 by detecting the code disk 19. When the magnet 161 rotates to the rotation origin, the photoelectric switch 18 can control the first motor 13 and/or the second motor 14 to stop working, so as to control the magnet 161 to stop rotating, thereby facilitating finding the rotation origin of the magnetic 161 to determine the initial position of the capsule endoscope, and to determine that the magnet 161 rotates for at least one circle, so that the capsule endoscope in vivo is driven to rotate for at least one circle by the variation of the magnetic field of the magnet 161, and that the capsule endoscope can observe the internal conditions of human stomach more completely.

Alternatively, in an embodiment, the rotation of the magnetic component 16 may also be controlled according to actual rotation circles of the magnet 161 (that is, the number of times that the magnet 161 penetrates through the rotation origin is detected). For example, a threshold value of the number of times that the photoelectric switch 18 detects that the magnet 161 penetrates the rotation origin can be defined. When it is detected that the number of times that the magnet 161 penetrates through the rotation origin exceeds the threshold value, the photoelectric switch 18 can control the first motor 13 and/or the second motor 14 to stop working. In an embodiment, the threshold value can be defined as 150, that is, the photoelectric switch 18 detects that the magnet 161 penetrates through the rotation origin 150 times.

In an embodiment, as shown in Figs. 6 and 7, the code disk 19 includes a first code disk 191, the photoelectric switch 18 includes a first photoelectric switch 181, and the detection portion includes a first detection portion provided at the first code disk 191. The first code disk 191 is installed at the lower base 122, the first photoelectric switch 181 is installed at the upper base 121, the first photoelectric switch 181 is provided with a first groove 181a, and the first photoelectric code disk 191 is provided with a first detection portion. When the first detection portion of the first code disk 191 is located in the first groove 181a, the first detection portion can cooperate with the first photoelectric switch 181 to control the start or stop of the circuit. That is, an initial rotation position of the first detection portion (when not powered on) is located in the first groove 181a, and when the first photoelectric switch 181 detects that the first detection portion is located in the first groove 181a, the magnet 161 is located at the first zero point, and the first code disk 191 can drive the first detection portion to rotate along with the lower base 122. When the first photoelectric switch 181 detects that the first code disk 191 is located in the first groove 181a again, the magnet 161 returns to the first zero point, and the first photoelectric switch 181 controls the first motor 13 to stop working. Once the first code disk 191 rotates for one circle, the first detection portion penetrates through the first groove 181a once.

In an embodiment, the first detection portion is a light barrier. The light barrier is provided at an outer edge of the first code disk 191 and can rotate along with the rotation of the magnet 161. In this embodiment, the upper base 121 is fixedly installed at the mounting base 11 and does not rotate. The first motor 13 controls the lower base 122 to rotate so as to drive the first code disk 191 and the magnet 161 to rotate. The first photoelectric switch 181 detects the rotation position of the magnet 161 by detecting a light barrier of the first code disk 191. When the light barrier of the first code disk 191 rotates to the first groove 181a again, the magnet 161 returns to the first zero point, and the light barrier shields light beams in the first groove 181a, so that the first photoelectric switch 181 detects the first code disk 191 and is automatically disconnected, and further, the first motor 13 is powered off and controlled to stop working, since the first photoelectric switch 181 is electrically connected to the power supply of the first motor 13.

Alternatively, in other embodiments of the present disclosure, the first detection portion may also include other structures as long as the magnet 161 can be controlled to stop at the rotation origin. For example, the first detection portion is an annular thin-wall light barrier provided at the outer edge of the first code disk 191, and the light barrier is provided with an opening or a transparent portion. When the opening or the transparent portion is located inside the first groove, the first photoelectric switch 18 can stop the magnet 161 at the rotation origin by controlling the first motor 13. In a specific application, according to the different types of photoelectric switch (for example, an opposite type photoelectric switch, a slot type photoelectric switch, etc.), the first detection portion that can cooperate with the photoelectric switch can be selected, as long as it can control the magnetic component 16 to stop exactly at the rotation origin, which will not be described in detail.

In an embodiment, as shown in Figs. 3 and 5, the housing 162 is rotatably installed at the lower base 122 through a shaft portion 162d. The code disk 19 further includes a second code disk 192. The photoelectric switch 18 further includes a second photoelectric switch 182. The second code disk 192 is installed at the housing 162 or the shaft portion 162d of the housing 162. The second photoelectric switch 182 is installed at the connecting plate 122a.The second photoelectric switch 182 is provided with a second groove 182a. The detection portion includes a second detection portion provided at the second code disk 192. When the second detection portion of the second code disk 192 is located in the second groove 182a, the second detection portion can cooperate with the second photoelectric switch 182 to control the circuit to start or stop. That is, the initial rotation position of the second detection portion (when not powered on) is located in the second groove 182a, the second code disk 192 drives the second detection portion to rotate with the housing 162. When the second photoelectric switch 182 detects that the second detection portion is located in the second groove 182a again, the magnet 161 returns to the rotation origin (the second zero point), and the second photoelectric switch 182 controls the second motor 14 to stop working. Once the second code disk 192 rotate for one circle, the second detection portion penetrates through the second groove 182a once.

In some embodiments of the present disclosure, the second detection portion may be an opening 192a. The opening 192a is provided at the edge of the second code disk 192 and can rotate along with the rotation of the magnet 161. In this embodiment, the housing 162 is rotatably connected to the connecting plate 122a, the second motor 14 controls the housing 162 to rotate, so as to drive the second code disk 192 and the magnet 161 to rotate. The second photoelectric switch 182 detects the rotation position of the magnet 161 by detecting the opening 192a of the second code disk 192. When the opening 192a of the second code disk 192 rotates into the second groove 182a again, the magnet 161 returns to the rotation origin (the second zero point), and the opening 192a allows the light beams pass through the second groove 182a, so that the second photoelectric switch 182 detects the opening 192a of the second code disk 192 and is automatically turned off, and further the second motor 14 is powered off and controlled to stop working, since the second photoelectric switch 182 is electrically connected to the power supply of the second motor 14.

Alternatively, in other embodiments of the present disclosure, the second detection portion may have other structures according to different types of photoelectric switch 18, as long as the magnet 161 can be controlled to stop at the second zero point, which will not be described in detail here.

It is worth mentioning that the switch assembly is a microswitch group, and the switch mating member is a contact mating portion. Along with a relative rotation between the microswitch group and the contact mating portion, when the microswitch group detects the contact mating portion, the circuit of the microswitch group is disconnected, and the magnetic component 16 stops at the rotation origin.

The microswitch group is electrically connected to the first motor 13 and/or the second motor 14, and includes a microswitch and a contact part. The microswitch is provided with a contact mating portion which can cooperate with the contact part, and the on/off of the circuit is controlled by the contact and separation of the contact part and the contact mating portion. The microswitch group is provided at the rotation origin of the magnetic control device 1, one of the contact part and the microswitch is configured to rotate along with the rotation of the magnet 161, and the other one of the contact part and the microswitch is fixed, and the two can cooperate with each other to control the on/off of the circuit, so that the magnetic control device 1 can stop at the rotation origin.

In order to adjust the first motor 13 and the second motor 14, respectively, at least two microswitch groups are provided to control the magnet 161 to stop at the first zero point and to control the magnet 161 to stop at the second zero point, respectively. In an embodiment, the microswitch groups (not shown in the figures) are provided at the upper base 121, the contact part (not shown in the figures) is an action reed, and the contact mating portion (not shown in the figures) is a bump provided at the lower base 122 and protruding toward the microswitch groups. The bump rotates along with the lower base 122. When the bump contacts the action reed, the microswitch controls the first motor 13 to be turned off, and the magnet 161 stops at the first zero point. In addition, the microswitch group is also provided at the lower base 122, and the bump is provided at the shaft portion 162d of the housing 162 of the magnetic component 16 and rotates along with the housing 162. When the bump contacts the action reed, the microswitch controls the second motor 14 to be turned off, and the magnet 161 stops at the second zero point, thereby facilitating an operator to use.

It should be noted that part of the patent application document contains the content protected by copyright. In addition to making copies of the patent documents of the Patent Office or recorded patent documents, the copyright is reserved by the copyright owner.

## Claims

1. A magnetic control device for controlling a capsule endoscope, comprising:
a mounting base configured to mount the magnetic control device;
a base connected to the mounting base;
a magnetic component, comprising a magnet configured to drive the capsule endoscope to rotate;
a first motor configured to control the magnetic component to rotate around a first axis, wherein the first motor penetrates through the mounting base and is installed at the base along a first direction of the magnetic control device; and
a second motor configured to control the magnetic component to rotate around a second axis, wherein the second motor is installed at the base;
wherein the first axis intersects the second axis.

2. The magnetic control device according to claim 1, wherein the base comprises an upper base and a lower base; and
the upper base is located between the mounting base and the lower base along the first direction of the magnetic control device, and a mounting space is defined between the mounting base and the lower base.

3. The magnetic control device according to claim 2, wherein an outer edge of the lower base exceeds an outer edge of the upper base.

4. The magnetic control device according to claim 2, wherein the first motor is installed at the upper base; and
the second motor is connected to the lower base and is provided in the mounting space between the mounting base and the lower base.

5. The magnetic control device according to claim 2, wherein the magnetic control device further comprises a mounting plate connected to the base; and
at least part of the mounting plate extends to the mounting space, and the second motor is connected with the base through the mounting plate.

6. The magnetic control device according to claim 2, wherein the magnetic control device further comprises a conductive slip ring comprising an upper slip ring and a lower slip ring; and
the upper slip ring is installed at the upper base and is electrically connected to a power supply, and the lower slip ring is installed at the lower base and is electrically connected to the second motor.

7. The magnetic control device according to claim 6, wherein the conductive slip ring is a printed circuit board (PCB) type slip ring, and a thickness of the PCB type slip ring is less than or equal to 10mm.

8. The magnetic control device according to anyone of claims 2-7, wherein the magnetic component comprises a housing; and
wherein the housing comprises a left housing and a right housing, the left housing is connected to the right housing through a flange, and the magnet is fixed in the housing.

9. The magnetic control device according to claim 8, wherein two connecting plates are provided at two ends of the lower base along a second direction of the magnetic control device, and the two connecting plates extend away from the upper base along the first direction of the magnetic control device; and
the housing is located between the two connecting plates and is rotatably connected to the two connecting plates, and the housing rotates under driven by the first motor and the second motor.

10. The magnetic control device according to claim 2, wherein the magnetic control device further comprises at least one rotation origin detection mechanism for determining whether the magnetic component is at a rotation origin; and
the rotation origin detection mechanism comprises:
a switch assembly electrically connected to the first motor and/or the second motor, and
a switch mating component cooperating with the switch assembly to control on/off of a circuit.

11. The magnetic control device according to claim 10, wherein the rotation origin detection mechanism is installed between the upper base and the lower base, the switch assembly is installed at one of the upper base and the lower base, and the switching mating component is installed at the other one of the upper base and the lower base; and wherein the first motor is controlled to start or stop by a relative rotation between the switch assembly and the switch mating component.

12. The magnetic control device according to claim 10, wherein the rotation origin detection mechanism is installed at a position where the lower base and the magnetic component are connected to each other, the switch assembly is installed at one of the lower base and the magnetic component, and the switch mating component is installed at the other one of the lower base and the magnetic component; and wherein the second motor is controlled to start or stop by a relative rotation between the switch assembly and the switch mating component.

13. The magnetic control device according to anyone of claims 10-12, wherein the switch assembly is a photoelectric switch, the switch mating component is a code disk, and the code disk is provided with a detection portion; and
along with a relative rotation between the photoelectric switch and the code disk, when the photoelectric switch detects the detection portion, the photoelectric switch controls the circuit to be disconnected, and the magnetic component stops at the rotation origin.

14. The magnetic control device according to anyone of claims 10-12, wherein the switch assembly is a microswitch group, and the switch mating component is a contact mating portion; and
along with a relative rotation between the microswitch group and the contact mating portion, when the microswitch detects the contact mating portion, the microswitch group controls the circuit to be disconnected, and the magnetic component stops at the rotation origin.

15. The magnetic control device according to claim 1, wherein the first motor and/or the second motor are/is a direct current motor or a stepper motor.

16. A magnetically controlled capsule endoscope system comprises the magnetic control device of any one of claims 1-15, wherein the magnetic control device is installed at the magnetically controlled capsule endoscope system through the mounting base.
